# EUROPEAN PATENT SPECIFICATION

(11) **EP 1 030 842 B1**
(45) Date of publication and mention of the grant of the patent: **02.04.2003**
(21) Application number: 97912351.0
(22) Date of filing: 13.11.1997
(51) Int. Cl.: C07D 213/75, A61K 31/44

(54) **METALLOPROTEINASE INHIBITORS**
METALLOPROTEINASE INHIBITOREN
INHIBITEURS DE METALLOPROTEINASE

(43) Date of publication of application: 30.08.2000
(73) Proprietor: BRITISH BIOTECH PHARMACEUTICALS LIMITED, Cowley Oxford, OX4 5LY (GB)
(72) Inventor: BECKETT, Raymond, Paul, Watlington Road Cowley Oxford OX4 5LY (GB); WHITTAKER, Mark, Watlington Road Cowley Oxford OX4 5LY (GB); MILLER, Andrew, Watlington Road Cowley Oxford OX4 5LY (GB); MARTIN, Fionna, Mitchell, Watlington Road Cowley Oxford OX4 5LY (GB)
(74) Representative: Walls, Alan James
(86) International application number: GB9703132
(87) International publication number: WO99025693

(56) References cited:
- WO-A-95/19956
- WO-A-97/03966

## Description

The present invention relates to N¹-[2,2-Dimethyl-1S-(pyridin-2-ylcarbamoyl)-propyl]-N⁴-hydroxy-2R-isobutyl-3S-methoxy-succinamide, and its pharmaceutically acceptable salts, hydrates and solvates.

### Background to the Invention

International patent application WO 95/19956 (British Biotech Pharmaceuticals Ltd) describes and claims a class of compounds which are matrix metalloproteinase (MMP) inhibitors and inhibitors of the release of tumour necrosis factor (TNF-α) from cells. Specifically, the application claims compounds of general formula (I) 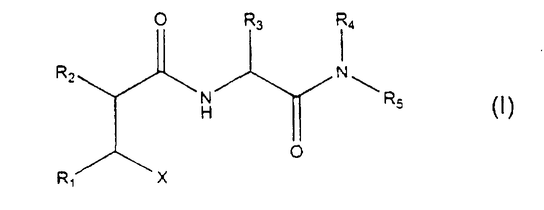 wherein
X is a -CO₂H or -CONHOH group;
R₁ is hydrogen; (C₁-C₆)alkyl; (C₂-C₆)alkenyl; phenyl; substituted phenyl; phenyl (C₁-C₆)alkyl); substituted phenyl(C₁-C₆)alkyl; heterocyclyl; substituted heterocyclyl; heterocyclyl(C₁-C₆)alkyl; substituted heterocyclyl(C₁-C₆)alkyl; a group BSOₙA-wherein n is 0, 1 or 2 and B is hydrogen or a (C₁-C₆) alkyl, phenyl, substituted phenyl, heterocyclyl, (C₁-C₆)acyl, phenacyl or substituted phenacyl group, and A represents (C₁-C₆)alkyl; amino; protected amino; acylamino; OH; SH; (C₁-C₆)alkoxy; (C₁-C₆)alkylamino; di-(C₁-C₆)alkylamino; (C₁-C₆)alkylthio; aryl (C₁-C₆)alkyl; amino(C₁-C₆)alkyl; hydroxy(C₁-C₆)alkyl, mercapto(C₁-C₆)alkyl or carboxy(C₁-C₆)alkyl wherein the amino-, hydroxy-, mercapto- or carboxyl-group are optionally protected or the carboxyl- group amidated; lower alkyl substituted by carbamoyl, mono(lower alkyl)carbamoyl, di(lower alkyl)carbamoyl, di(lower alkyl)amino, or carboxy-lower alkanoylamino;
R₂ is a (C₁-C₆)alkyl, (C₂-C₆)alkenyl, (C₂-C₆)alkynyl, phenyl(C₁-C₆)alkyl, heteroaryl(C₁-C₆)alkyl, cycloalkyl(C₁-C₆)alkyl or cycloalkenyl(C₁-C₆) alkyl group, any one of which may be optionally substituted by one or more substituents selected from (C₁-C₆)alkyl, -O(C₁-C₆)alkyl, -S(C₁-C₆)alkyl, halo and cyano (-CN);
R₃ is the characterising group of a natural or non-natural α amino acid in which any functional groups may be protected;
R₄ is a phenyl or 5- or 6-membered heteroaryl ring wherein any ring nitrogen atom may be oxidised as an N-oxide, which may be optionally fused to a benzene ring or to a 5-, 6- or 7-membered heterocyclic ring, and wherein any of the rings may be optionally substituted by:
   (a) one or more substituents independently selected from hydroxyl, halogen, -CN, -CO₂H, -CO₂(C₁-C₆)alkyl, -(C₁-C₆)alkyl-CO₂(C₁-C₆)alkyl, -CONH₂, - CONH(C₁-C₆)alkyl, -CON((C₁-C₆)alkyl)₂, -CHO,
      - CH₂OH, -(C₁-C₄)perfluoroalkyl, -O(C₁-C₆)alkyl, -S(C₁-C₆)alkyl,
      - SO(C₁-C₆)alkyl, -SO₂(C₁-C₆)alkyl, -NO₂, -NH₂, -NH(C₁-C₆)alkyl,
      - N((C₁-C₆)alkyl)₂, and -NHCO(C₁-C₆)alkyl, or
   (b) a group selected from (C₁-C₆)alkyl, (C₂-C₆)alkenyl, (C₂-C₆)alkynyl, (C₃-C₈)cycloalkyl, (C₄-C₈)cycloalkenyl, phenyl, benzyl, heteroaryl or heteroarylmethyl any of which groups may be optionally substituted with one or more substituents selected from halogen, hydroxyl, amino, carboxyl, (C₁-C₄)perfluoroalkyl, (C₁-C₆)alkyl, -O(C₁-C₆)alkyl or - S(C₁-C₆)alkyl;
R₅ is hydrogen or a (C₁-C₆)alkyl group;
or a salt, hydrate or solvate thereof.

International Patent Application WO 97/03966 discloses additional compounds of the class first disclosed in the above WO 95/19956.

### Brief description of the Invention

N¹-[2,2-Dimethyl-1S-(pyridin-2-ylcarbamoyl)-propyl]-N⁴-hydroxy-2R-isobutyl-3S-methoxy-succinamide is a member of the class generically disclosed and claimed in WO 95/19956, but neither it nor its properties are otherwise particularised.

WO 95/19956 states that in the disclosed class of compounds the aromatic or heteroaryl R₄ substituent has in general the unexpected and desirable effect of increasing activity against stromelysin relative to known compounds of otherwise similar structure but with the different (usually methyl) R₄ substituents present in such known compounds, while maintaining activity against collagenase and gelatinase. As a member of the class, the now selected compound N¹-[2,2-dimethyl-1S-(pyridin-2-ylcarbamoyl)-propyl]-N⁴-hydroxy-2R-isobutyl-3S-methoxy-succinamide posesses these properties.

However, it is known that a side effect of some MMP inhibitors is induction of musculo-skeletal pain (sometimes also referred to as "tendinitis") in the joints, for example the shoulder, in some animals and patients after high and/or prolonged dosing. Although it is believed that this effect is substantially reversable on cessation of dosing, it is nevertheless undesirable. The mechanism by which the pain arises is not presently understood, and it has not so far proved possible to correlate the propensity of the compound to induce pain with particular structural features or enzyme inhibition profile of the molecule. Accordingly, whether any given MMP inhibitor will cause this side effect, and the severity of the effect if any, cannot presently be predicted and must be tested empirically.

It has now been found that the selected N¹-[2,2-dimethyl-1S-(pyridin-2-ylcarbamoyl)-propyl]-N⁴-hydroxy-2R-isobutyl-3S-methoxy-succinamide, has a very much reduced tendency to induce the musculo-skeletal pain side effect. In this respect it differs from close structural analogues, for example N¹-[2,2-dimethyl-1S-(pyridin-3-ylcarbamoyl)-propyl]-N⁴-hydroxy-2R-isobutyl-3S-hydroxy-succinamide,
which are more prone to induce that side effect.

WO 95/19956 also states that the disclosed class of arylamide MMP inhibitors includes compounds which are orally bioavailable. Not all compounds encompassed by the disclosure of WO 95/19956 are orally bioavailable to a useful degree, as evidenced for example by the peak level of MMP inhibitory activity or level of activity over time ("area under the curve") attributable to the drug in the blood of animals orally dosed with the drug. It has been found that the compound selected according to the present invention, N¹-[2,2-dimethyl-1S-(pyridin-2-ylcarbamoyl)-propyl]-N⁴-hydroxy-2R-isobutyl-3S-methoxy-succinamide, is orally bioavailable in humans and other mammals.

This combination of oral bioavailability and reduced liability to tendinitic side effects implies that the compound of the invention should offer a relatively wide therapeutic window for treatment of diseases requiring medium or longer term systemic administration of an MMP inhibitor. The compound therefore is indicated for treatment of, for example, rheumatoid arthritis, cancers, multiple sclerosis (MS), Guillain-Barre syndrome (GBS) and psoriasis.

### Detailed Description of the Invention

The present invention therefore provides N¹-[2,2-dimethyl-1S-(pyridin-2-ylcarbamoyl)-propyl]-N⁴-hydroxy-2R-isobutyl-3S-methoxy-succinamide, of formula (II) 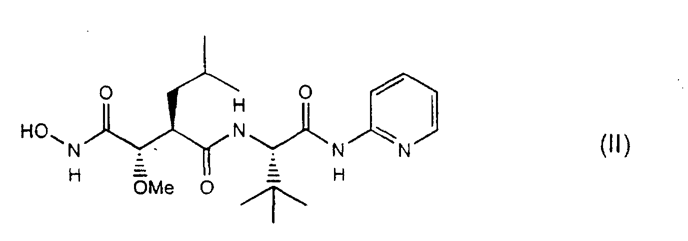 and its pharmaceutically acceptable salts, hydrates and solvates.

The compound of the invention has three asymmetric carbon atoms, whose stereochemical configuration is as specified in the name of the compound and shown in formula (II). However, it is to be understood that the invention includes mixtures of the enantiomers of the compound (II), provided the specified enantiomer predominates. Preferably, 90% or more by weight of such enantiomeric mixtures should be the enantiomer specified.

The invention also includes pharmaceutical compositions comprising N¹-[2,2-dimethyl-1S-(pyridin-2-ylcarbamoyl)-propyl]-N⁴-hydroxy-2R-isobutyl-3S-methoxy-succinamide or a pharmaceutically acceptable salt, hydrate or solvate thereof, together with a pharmaceutically acceptable carrier. Preferred compositions of the invention are those which are adapted for oral administration.

The invention further includes the use of N¹-[2,2-dimethyl-1S-(pyridin-2-ylcarbamoyl)-propyl]-N⁴-hydroxy-2R-isobutyl-3S-methoxy-succinamide or a pharmaceutically acceptable salt, hydrate or solvate thereof, in the preparation of a pharmaceutical composition for treatment of rheumatoid arthritis, cancers, multiple sclerosis (MS), Guillain-Barre syndrome (GBS) or psoriasis in mammals, including humans.

Salts of the compound of the invention include physiologically acceptable acid addition salts for example the hydrochloride, hydrobromide, sulphate, methane sulphonate, p-toluenesulphonate, phosphate, acetate, citrate, succinate, lactate, tartrate, fumarate and maleate. Salts may also be formed with bases, for example sodium, potassium, magnesium, and calcium salts.

The selected compound of the invention may be prepared by the route described in the Example herein, and may be presented for administration by any route consistent with its pharmacokinetic properties. Orally administrable compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents; fillers; tabletting lubricant; disintegrants; or acceptable wetting agents. Tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents; emulsifying agents; non-aqueous vehicles (which may include edible oils); preservatives; and if desired conventional flavouring or colouring agents.

The active ingredient may also be administered parenterally in a sterile medium. Depending on the vehicle and concentration used, the drug can either be suspended or dissolved in the vehicle. Adjuvants such as a local anaesthetic, preservative and buffering agents can be dissolved in the vehicle.

The dose of the compound of the invention for any given clinical indication by a given route of administration will be determinable by clinical trials in accordance with standard practice for the granting of regulatory approval by the relevant authorities. In general it is presently expected that the compound will be administered orally to humans in dosage units of from 5 to 100 mg, twice or three times daily.

The following Example describes the preparation of the compound of the invention. The starting materials 2R-(2,2-Dimethyl-5-oxo-[1,3]dioxolan-4S-yl)-4-methyl-pentanoic acid and L*-tert*-leucine-N-(2-pyridyl)amide were prepared as described in WO 95/19961.

The following abbreviations have been used in the Example:
- DMF: N,N-Dimethylformamide
- EDC: N-Ethyl-N'-(3-dimethylaminopropyl)carbodiimide hydrochloride
- HOBt: 1-Hydroxybenzotriazole
- NMM: N-Methylmorpholine
- THF: Tetrahydrofuran

EXAMPLE

N¹-[2,2-Dimethyl-1S-(pyridin-2-ylcarbamoyl)-propyl]-N⁴-hydroxy-2R-isobutyl-3S-methoxy-succinamide

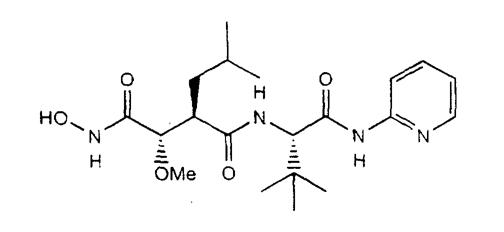

### STEP A:

2S-Hydroxy-3R-isobutyl-succinic acid dimethyl ester

2R-(2,2-Dimethyl-5-oxo-[1,3]dioxolan-4S-yl)-4-methyl-pentanoic acid (75.0 g, 0.326 mmol) was dissolved in methanol (500 ml) and cooled to 0°C and the resulting solution was saturated with hydrogen chloride gas. The reaction mixture was allowed to warm to room temperature and stirred overnight. The solvent was removed under reduced pressure and the residue was dissolved in dichloromethane and washed successively with sat. sodium hydrogen carbonate and brine. The organic phase was dried over anhydrous magnesium sulphate, filtered and evaporated to dryness under reduced pressure to give the title compound as a yellow oil (53 g, 75%). ¹H-NMR; δ (CDCl₃), 4.10 (1H, d, J = 4.0 Hz), 3.60 (3H, s), 3.50 (3H, s), 3.18 (brs), 2.78 (1H, m), 1.61 - 1.40 (2H, m), 1.28 (1 H, m) and 0.76 - 0.73 (6H, m).STEP B:

2R-Isobutyl-3S-methoxy-succinic acid dimethyl ester

2S-Hydroxy-3R-isobutyl-succinic acid dimethyl ester (23.9 g, 110 mmol) was dissolved in DMF (200 ml) and distilled iodomethane (8.2 ml, 132 mmol) was added, followed by silver (I) oxide (27.95 g, 121 mmol). The reaction was stirred for 7 days at room temperature with the exclusion of light. The solvent was removed under reduced pressure and the residue was purified by column chromatography (silica gel, dichloromethane as eluent) to give the title compound as a viscous yellow oil (19.16 g. 75%). ¹H-NMR; δ (CDCl₃), 3.83 (1H, d, J = 7.5 Hz), 3.71 (3H, s), 3.62 (3H, s), 3.30 (3H, s), 2.85 (1H, m), 1.65 - 1.39 (2H, m), 1.10 (1H, m) and 0.83 - 0.81 (6H, m).

### STEP C:

2R-lsobutyl-3S-methoxy-succinic acid dilithium salt

Lithium hydroxide (1.76 g, 42.0 mmol) was added to a solution of 2R-isobutyl-3S-methoxy-succinic acid dimethyl ester (4.70 g, 20.0 mmol) in methanol (30 ml) and water (30 ml). The reaction mixture was stirred at room temperature for 2 hours then the solvents were removed under reduced pressure to give the title compound as a yellow solid (4.40 g, quant.). ¹H-NMR; δ (CD₃OD), 3.52 (1H, d, J = 5.1 Hz), 3.27 (3H, s), 2.65 (1H, m), 1.56 - 1.53 (2H, m), 1.31 (1H, m) and 0.82 - 0.78 (6H, m).

### STEP D:

2R-Isobutyl-3S-methoxy-succinic acid 4-methyl ester

2R-lsobutyl-3S-methoxy-succinic acid dilithium salt (25.14 g, 116 mmol) was dissolved in dry THF (150 ml) and the solution was cooled to 0°C. Trifluoroacetic anhydride (30 ml) was added and the mixture was stirred at 0°C for a further 4 hours. The solvent was removed under reduced pressure and the residue was dissolved in anhydrous methanol (200 ml) at 0°C and the solution was stirred overnight at room temperature. The solvent was removed under reduced pressure to give the title compound as a yellow oil (54.3 g, including 2 equivalents of lithium trifluoroacetate), which was used without further purification in STEP E. ¹H-NMR; δ (CD₃OD), 7.71 (1H, d, J = 7.5 Hz), 3.65 (3H, s), 3.24 (3H, s), 2.72 (1H, m), 1.56 - 1.42 (2H, m), 1.06 (1H, m) and 0.81 - 0.79 (6H, m).

### STEP E:

3R-[2,2-Dimethyl-1S-(pyridylcarbamoyl)-propylcarbamoyl]-2S-methoxy-5-methyl-hexanoic acid methyl ester.

The product from STEP D (25.06 g, equivalent to 53.7 mmol 2R-isobutyl-3S-methoxy-succinic acid 4-methyl ester) was dissolved in DMF (200 ml) and the solution was cooled to 0°C during the addition of HOBt (8.70 g, 53.7 mmol) followed by EDC (12.35 g, 64.4 mmol). The mixture stirred and allowed to warm to room temperature over 2 hours. The solution of active ester thus formed was cooled to 0°C, L-*tert*-leucine-N-(2-pyridyl)amide (11.11 g, 53.7 mmol) was added and the mixture was stirred overnight at room temperature. The solvent was removed under reduced pressure and the residue was dissolved in ethyl acetate. The solution was washed successively with 1M sodium carbonate solution, and brine, dried over anhydrous magnesium sulphate, filtered and evaporated to dryness. The residue was purified by column chromatography (silica gel, 0 to 5% methanol in dichloromethane) to afford the title compound as a white solid (13.41 g, 61%). ¹H-NMR; δ (CDCl₃), 9.61 (1H, s), 8.47 (1H, m), 8.24 (1H, d, J = 8.4 Hz), 7.74 (1H, m), 7.07 (1H, m), 6.97 (1H, d, J = 8.9 Hz), 4.64 (1H, d, J = 8.9 Hz), 4.01 (1H, d, J = 7.6 Hz), 3.76 (3H, s), 3.41 (3H, s), 2.75 (1H, m), 1.79 (1H, m), 1.51 (1H, m), 1.11 (1H, m), 1.02 (9H, s), 0.84 (3H, d, J = 6.3 Hz), and 0.82 (3H, d, J = 6.3 Hz).

### STEP F:

### 3R-[2,2-Dimethyl-1S-(pyridin-2-ylcarbamoyl)-propylcarbamoyl]-2S-methoxy-5-methyl-hexanoic acid lithium salt

3R-[2,2-Dimethyl-1S-(pyridin2-ylcarbamoyl)-propylcarbamoyl]-2S-methoxy-5-methyl-hexanoic acid methyl ester (13.4 g, 32.9 mmol) was dissolved in a mixture of THF (265 ml) and water (65 ml) and lithium hydroxide monohydrate (1.396 g, 33.3 mmol) was added. The solution was stirred at room temperature for 2 hours then evaporated under reduced pressure to provide a yellow oil which was dried further by azeotroping with toluene. The product (13.4 g, containing residual solvent) was used immediately without further purification. ¹H-NMR; δ (CD₃OD, 3.5:1 mixture of diastereoisomers), 8.31 (1H, m), 7.99 (1H, d, J = 8.3 Hz), 7.66 (1H, m), 7.00 (1H, m), 4.49 (0.23H, s; minor diastereoisomer), 4.37 (0.77H, s, major diastereoisomer), 3.68 (0.23H, d, J = 7.6 Hz; minor diastereoisomer), 3.52 (0.77H, d, J = 7.6 Hz; major diastereoisomer), 3.21 (0.69H, s; minor diastereoisomer), 3.20 (2.31H, s, major diastereoisomer), 2.64 (1H, m), 1.53 (2H, br m), 1,18 (1H, m), 1.01 (9H, s), 0.85 (3H, d, J = 6.4 Hz) and 0.81 (3H, d, J = 6.3 Hz).

### STEP G:

### N⁴-Benzyloxy-N¹-[2,2-dimethyl-1S-(pyridin-2-ylcarbamoyl)-propyl]-2R-isobutyl-3S-methoxy-succinamide

The product from STEP F (13.4 g, ca. 33 mmol) was dissolved in dry DMF (250 ml) and placed under argon and cooled to -10°C with stirring. Ethyl chloroformate (3.47 ml, 36 mmol) was added dropwise, followed by NMM (1.8 ml, 16.5 mmol). The mixture was stirred for 30 minutes before dropwise addition of O-benzylhydroxylamine (6 g, 49 mmol) in DMF (10ml). The reaction mixture was allowed to warm to room temperature and stirred overnight. The solvent was removed under reduced pressure and the residue was partitioned between ethyl acetate and brine. The organic layer was washed with 1M sodium carbonate solution, dried over magnesium sulphate, filtered and evaporated. The residue was purified by flash chromatography (silica gel, 5% methanol in dichloromethane). Fractions containing the desired product were pooled and evaporated. The product was triturated with diethyl ether to remove slight coloured impurity. Yield: 9.44 g (58%, >10:1 mixture of diastereoisomers). ¹H-NMR; δ (CDCl₃ major diastereoisomer), 10.26 (1H, s), 9.93 (1H, s), 8.32 (1H, m), 8.23 (1H, d, J = 8.2 Hz), 7.63 (1H, m), 7.25 (5H, m), 7.12 (1H, d, J = 9.2 Hz), 7.02 (1H, m), 4.94 (1H, d, J = 10.8 Hz), 4.76 (2H, d, J = 3.8 Hz), 3.88 (1H, d, J = 5.5 Hz), 3.32 (3H, s), 2.91 (1H, m), 1.72 (1H, m), 1.55 (1H, m), 1.35 (1H, m), 1.02 (9H, s), 0.89 (3H, d, J = 6.5 Hz) and 0.85 (3H, d, J = 6.5Hz).

### STEP H:

### N¹-[2,2-Dimethyl-1S-(pyridin-2-ylcarbamoyl)-propyl]-N⁴-hydroxy-2R-isobutyl-3S-methoxy-succinamide

N⁴-Benzyloxy-N¹-[2,2-dimethyl-1S-(pyridin-2-ylcarbamoyl)-propyl]-2R-isobutyl-3S-methoxy-succinamide was dissolved in a mixture of methanol (75 ml) and ethanol (75 ml) and placed under an argon atmosphere. 10% palladium on charcoal was added and hydrogen gas was bubbled through the solution for 2 hours, at which time TLC analysis revealed that all of the starting material had been consumed. The system was purged with argon and the catalyst was removed by filtration. The solvents were removed under reduced pressure to provide the title compound as a white solid (8.8 g, quant.; 12:1 mixture of diastereoisomers). m.p. 163 - 164 °C. ¹H-NMR; δ ((CD₃)₂SO), 10.67 (1H, s), 10.13 (1H, s), 8.90 (1H, s), 8.15 (1H, m), 7.89 (1H, d, J = 8.4 Hz), 7.81 (1H, d, J = 8.8 Hz), 7.60 (1H, m), 6.93 (1H, m), 4.53 (0.12H, d, J = 9.4 Hz), 4.43 (0.88H, d, J = 8.8 Hz), 3.74 (0.12H, d, J = 10.0 Hz), 3.32 (0.88H, d, J = 9.8 Hz), 2.98 (0.3H, s), 2.96 (2.64H, s), 2.78 (1H, m), 1.23 (2H, m), 0.84 (10H, s and m), 0.65 (3H, d, J = 6.4 Hz) and 0.56 (3H, d, J = 6.3 Hz). ¹³C-NMR; δ (CD₃OD), 172.6, 170.0, 166.0, 151,5, 147.9, 136.0, 119.5, 113.6, 61.2, 60.6, 56.7, 46.2, 37.0, 34.0, 26.5, 25.2, 23.7 and 21.7. IR; vₘₐₓ (KBr), 3255, 2957, 1700, 1645, 1524, 1467, 1435, 1370, 1301, 1213 and 1152 cm⁻¹. Found: C 58.40, H 7.92, N 13.61%; C₂₀H₃₂N₄O₅ . 0.2H₂O requires C 58.29, H 7.92, N 13.60%.

### Biological Example A

The potency of the compound of the invention as inhibitors of interstitial collagenase was determined by the procedure of Cawston and Barrett, (Anal. Biochem., **99**, 340-345, 1979), whereby a 1mM solution of the compound being tested, or a dilution thereof, was incubated at 37° for 16 hours with collagen and collagenase (buffered with 25mM Hepes, pH 7.5 containing 5mM CaCl₂, 0.05% Brij 35 and 0.02% NaN₃). The collagen was acetylated ¹⁴C collagen prepared by the method of Cawston and Murphy, (Methods in Enzymology, **80**, 711, 1981), hereby incorporated by reference. The samples were centrifuged to sediment undigested collagen, and an aliquot of the radioactive supernatant removed for assay on a scintillation counter as a measure of hydrolysis. The collagenase activity in the presence of 1mM of the test compound, or a dilution thereof, was compared to activity in a control devoid of inhibitor and the result reported below as that of inhibitor concentration effecting 50% inhibition of the collagenase activity (IC₅₀).

The potency of the compound of the invention as inhibitors of stromelysin-1 was determined by the procedure of Cawston et al, (Biochem. J., **195**, 159-165, 1981), whereby a 1mM solution of the compound being tested, or a dilution thereof, was incubated at 37° for 16 hours with stromelysin and ¹⁴C acetylate casein (buffered with 25mM Hepes, pH 7.5 containing 5mM CaCl₂, 0.05% Brij 35 and 0.02% NaN₃). The casein was acetylated ¹⁴C casein prepared by the method of Cawston et al (ibid). The stromelysin activity in the presence of 1 mM of the test compound, or a dilution thereof, was compared to activity in a control devoid of inhibitor and the result reported below as that of inhibitor concentration effecting 50% inhibition of the stromelysin activity (IC₅₀).

The potency of the compound of the invention as inhibitors of 72 kDa gelatinase was determined by a procedure based on the method of Sellers et. al, Biochem. J., **171**, 493-496 (1979). 72 kDa gelatinase, derived from RPMI-7951 cells was purified by gelatin-agarose chromatography. The enzyme was activated by incubation with aminophenyl mercuric acetate and approximately 0.05 units was incubated with 50µg [¹⁴C]-radiolabelled gelatin in an appropriate buffer for 16 hours at 37'C. At the end of the incubation 50µg bovine serum albumin, together with trichloroacetic acid (final concentration 16%) were added to stop the reaction and to precipitate any undegraded substrate. The reaction tubes were placed on ice for 15 minutes before centrifugation at 10,000g for 15 minutes to sediment the precipitated substrate. A 200µl aliquot of the reaction supernatant was removed and the radioactivity determined by liquid scintillation counting. The effect of the inhibitors was determined by reference to a dose response curve. The IC₅₀ (the concentration of inhibitor required to cause a 50% decrease in enzyme activity) was obtained by fitting a curve to the data and computing the concentration of inhibitor required to achieve 50% inhibition of the enzyme. For each IC₅₀ determination, the effect on gelatinase activity of at least 8 concentrations of the inhibitor were examined. The inhibitors were dissolved and diluted in DMSO.

The results of the above tests with the compound of the invention were as follows:

| Enzyme | IC₅₀ (nM) |
|---|---|
| Collagenase | 10 |
| 72 kDa gelatinase | 70 |
| Stromelysin | 30 |

### Biological Example B

The concentration over time of compound of the invention in the blood of laboratory animals following administration of the test compounds was measured. The compound was administered by gavage to 6 male rats (300g) per treatment group. Blood samples were removed by tail venepuncture at 0.5, 1.0, 2.0, 6.0 and 24 hours post administration. 0.4 ml of blood was placed into 4.5ml tubes containing 0.1ml acid citrate dextrose (ACD) as anti-coagulant. For extraction, 3ml methanol was added and the precipitated blood pelletted by centrifugation (30 min at 3000 rpm). A 2ml aliquot of supernatant was removed and concentrated by lyophilisation. The extract was redissolved in 200µl DMSO and a 10µl aliquot assayed for collagenase inhibitory activity. The inhibitory activity in the extracts was determined using the collagenase assay described in Biological Example A above, and the concentration of inhibitor (that is drug plus any active metabolites) obtained by comparison with standard curves. Results are expressed as peak concentration in ng/ml, as area under the curve (AUC) in ng/ml x hours, over 0.5-24 hours, and as AUC in number of IC₅₀'s x hours.

| **Results** | | |
|---|---|---|
| Peak conc | AUC (0.5 - 24h) | AUC (0.5 - 24h) |
| ng/ml | ng/ml x h | n IC₅₀'s x h |
| 212 | 2966 | 674 |

The compound of the invention has also been tested by oral administration to marmosets and healthy human volunteers and significant levels of inhibitory activity have been detected in the blood of those subjects after such administration.

### Biological Example C

The compound of the invention was tested in two animal models of cancer, and found to be active:

### B16 mouse melanoma model

In this model C57/BL6J mice are inoculated subcutaneously with 10⁵ B16 mouse melanoma cells. The tumours are allowed to grow over 18 days and the tumour weight calculated by calliper measurements and the following formula: weight (mg)- length (mm) x width (mm) ö 4. A group of mice (n=19) received the compound, administered via a mini-osmotic pump implanted subcutaneously on the opposite flank to the B16 tumour. The pump was implanted the day before tumour inoculation and the compound was delivered at a dose of 360 µg/day. A control group (n=17) received the appropriate volume of vehicle from identical implanted pumps.

The mean weight of the control tumours at day 18 was 1145 ± 97 mg (Figure 15). The weight of the compound-treated tumours was 774 ± 50 mg. This reduction (32% for the test compound) was significant (p<0.005). Assessment of samples at the end of the study indicated that plasma level was 26.8 ± 3.2 ng/ml for the test compound.

### MDA-435 breast carcinoma model

In this model, animals were inoculated with MDA-435 cells (10⁶ cells/mouse), the tumours allowed to grow for four days and then the animals were randomised by tumour weight into three treatment groups (n=15 per group). Mini-pumps were surgically implanted on day 5 containing either vehicle or test compound at 15 mg/ml or 60 mg/ml (delivering 180 or 720 µg/mouse/day respectively). The pumps were replaced on day 19 and the study ended on day 32. There was a dose-related inhibition of tumour growth, with 19% inhibition at 180 µg/mouse/day and 33% at 720 µg/mouse per day. The inhibition at the higher dose was statistically significant (p<0.005). Assessment of samples at the end of the study indicated that plasma levels were 99 ± 6 ng/ml and 136 ± 42 ng/ml for the low dose and high dose groups respectively.

### Biological Example D

The compound of the invention was tested for its propensity to induce observable signs of tendinitis in rats. The tendinitic effect in rats of the compound of the invention ("Compound A") was compared with that of a close structural isomer, namely N¹-[2,2-dimethyl-1S-(pyridin-3-ylcarbamoyl)-propyl]-N⁴-hydroxy-2R-isobutyl-3S-hydroxy-succinamide ("Compound B").

Male Lewis rats were used. In each study up to 30 rats were weighed and randomised on bodyweight for group allocation, n=6/group. In each study, a corresponding vehicle group was run to compare with each treatment group.

Compound A was formulated as its mesylate salt, whilst Compound B was formulated as the free base. For Compound A @ 15mg/ml the vehicle was 50% DMSO, 37% 0.1M methane sulphonic acid, 13% sterile water; and @ 30mg/ml the vehicle was 50% DMSO, 37% 0.2M methane sulphonic acid, 13% sterile water. For Compound B @ 15mg/ml the vehicle was 50% DMSO/water.

Alzet (trade mark) osmotic minipumps (2ML2, 14 day, 5µl/hr from Alza Corp., Palo Alto CA 94303), were weighed empty and full to be sure of correct fill volume. Prior to implantation, filled pumps were placed in an incubator at 37°C. All rats were anaesthetized with halothane. Once anaesthetised, the scruff was shaved and disinfected. A longitudinal skin incision, approx 2 cm long, was made on the prepared site and a subcutaneous pocket was made under the skin using a pair of haemostatic forceps. Minipumps were inserted with the exit of the pump facing away from the wound. The incision wound was closed with sutures, and the area around the wound was re-disinfected. Immediately post-operatively, all the rats received analgesic, (Temgesic -trade mark - Reckitt and Colman) 0.1mg/kg s.c. in the flank. On recovery from anaesthesia, the animals were returned to cages containing dry bedding to ensure the wound remained dust free prior to healing. On the day followng surgery, all rats were placed back on standard bedding and housed in groups of 3 to enable more accurate observation of signs of tendinitis.

Following the implantation of mini-pumps, rats were weighed and monitored for the possible onset of tendinitis. Onset and severity of tendinitis was measured by an observation based scoring system (see below). The rats were observed daily for 16 days. Mean scores >2 were considered significant. The scoring system used was as follows:

| Reclining: | | When stimulated to move animals showed | |
|---|---|---|---|
| Normal | 0 | Normal movement | 0 |
| Resting on one foot | 1 | Reluctance to move | 1 |
| Resting on no feet | 2 | Moderate reluctance to move | 2 |
| | | Very reluctant to move | 3 |

| Gait: | |
|---|---|
| Normal | 0 |
| Avoiding use of 1 hind foot | 1 |
| Avoiding use of both hind feet | 2 |

The results showed that the rats dosed with Compound A (at both 15mg/ml and 30mg/ml, and vehicle only, exhibited no significant signs of tendinitis, whereas those dosed with compound B showed significant signs from day 8 onwards (mean scores of >2 on day 8 rising to about 6 on days 15 and 16.

It was confirmed that rats dosed with Compounds A and B in the above tests were receiving plasma exposure to the compounds from the minipumps. Blood samples were obtained on days 3 and 10 post minipump implantation from rats lightly anaesthetised with halothane via the tail vein. Blood samples (0.5ml) were placed into tubes containing 3.0ml methanol to extract free and bound compound. Blood concentration of Compound A or Compound B was determined by a fluorometric assay using the coumarin peptide substrate Mca-Pro-Leu-Gly-Leu-Dpa-Ala-Arg-NH₂ (Mca = (7-methoxycoumarin-4-yl)acetyl, Dpa = N-3-(2,4-dinitrophenyl)-L-2,3-diaminopropyl) (see Knight et. al., FEBS Lett. 1992, 296, 263-266). On either day 16 or 17 post implantation, studies were ended, rats were terminally anaesthetised with halothane and blood samples (0.5ml) were taken by cardiac puncture and the blood concentration of Compound A or Compound B determined.

### Biological Example E

The effect of the compound of the invention was tested in an animal model of GBS Experimental autoimmune neuritis (EAN) is a T cell mediated, autoimmune disorder of the peripheral nervous system. The model exhibits many pathological features of GBS, with symptoms of ataxia, weakness and paralysis. EAN can be induced when animals are injected with myelin from peripheral nerves, or with protein components of the myelin such as Protein 0, in adjuvant. EAN lesions occur in the spinal roots and peripheral nerves and are characterised by perivascular mononuclear cell infiltration, demyelination of axons and nerve conduction deficit. TNFα has been implicated in the pathology of GBS and EAN; TNFα levels are elevated in the blood of GBS patients and anti-TNFα antibodies reduce disease severity in EAN (Hartung HP. Annals of Neurology 1993;33:563-567.).

The compound of the invention was tested in a rat EAN model where disease symptoms were induced by injection of peripheral nerve myelin in adjuvant.

The compound was administered from surgically-implanted minipumps at 15 or 30 mg/ml at a rate of 5 µl/hour (see Biological Example D), delivering 7 and 14 mg/kg/day respectively. Treatment with the compound throughout the course of the experiment from day 1-15 significantly reduces the development of clinical symptoms in a dose-dependent manner. The compound also reduces clinical symptoms in a dose dependent manner in the EAN model when given therapeutically from day 8-15 at 15 or 30 mg/ml at a rate of 10 µl/hour, delivering 14 and 28 mg/kg/day respectively.

### Biological Example F

The activity of the compound of the invention in an experimental model of MS:

### Method

The delayed type hypersensitivity model of MS described by Matyszak and Perry was used (Matyszak MK & Perry VH. 1995. Demyelination in the central nervous system following a delayed type hypersensitivity response to bacillus Calmette-Guerin. Neuroscience 64:967-977). Male Lewis rats were anaesthetised and given an injection of a 1µl phosphate buffered saline solution containing 10⁵ heat killed bacillus Calmette-Guerin (BCG) organisms into the striatum of the left hemisphere. BCG was injected stereotaxically via a 27G, 10µl capacity syringe. The coordinates for injection were; bregma +1.2mm, lateral +3.0mm and depth 4.5mm. After 4 weeks, 200µl of a solution containing 10⁷ heat killed BCG organisms in complete Freunds adjuvant was injected intradermally into the hind limb. After an additional 15 days rats received an intravenous injection of 2750U of type II horseradish peroxidase (HRP). Thirty minutes later rats were deeply anaesthetised with pentobarbitone sodium and transcardially perfused with 100ml of 0.9% (w/v) NaCI containing 5000U/L heparin, followed by 200ml of parformaldehyde-lysine-periodate fixative (PLP) containing 2%paraformaldehyde and 0.05% glutaraldehyde. The brains were removed, post-fixed for a further 4 hours in PLP and cryoprotected by immersion in 30% sucrose overnight at 4°C before being embedded in Tissue-Tek O.C.T. (miles inc Elkhart USA) and frozen in liquid nitrogen. Coronal free-floating 50um thick sections were cut for HRP localisation by the Hanker-Yates method (Perry VH et al 1992). This procedure generates a delayed-type hypersensitivity reaction at the site of the stereotaxic injection of BCG characterised by: local breakdown of the blood-brain barrier as indicated by staining for the extravascular presence of HRP; infiltration of lymphocytes as indicated by staining with the antibody OX-22, specific for the high molecular weight form of the leucocyte common antigen; activation of leucocytes as indicated by staining with the antibody OX-6, specific for class II major histocompatibility antigen; and myelin breakdown as indicated by staining with an antibody to myelin basic protein. These features are all hallmarks of the active lesions or plaques seen in the central nervous sytem of patients with MS. Lymphocytes were enumerated by counting the number of OX-22 positive cells in the region where staining was most intense. The number of cells in a single field of view was recorded and expressed as cells/mm². The area of MHC class II expression, blood brain barrier leakage and demyelination were calculated using computer aided image analysis and output data expressed as mm².

The effects of the compound of the invention were assesed by treating rats with 30mg/kg given orally twice daily beginning 5 days after the second injection of BCG and continuing until day 15. The compound of the invention was formulated in a phosphate buffered saline vehicle containing 0.01% Tween 80. Animals as controls received the vehicle only.

The areas of blood-brain barrier leakage, MHC class II expression, demyelination and numbers of T cells in animals treated with the compound of the invention were compared to vehicle treated controls using Students T test.

### Results

In vehicle treated animals the DTH response was characterised by the breakdown of the blood-brain barrier, infiltration of lymphocytes, MHC class II expression and demyelination. In animals treated with the compound of the invention there were significant reductions in the area of blood-brain barrier leakage(p<0.05) and in the numbers of infiltrating T cells (p<0.0001). Reductions in the area of demyelination and MHC class II expression were observed, but did not reach statistical significance.

The effect of the compound of the invention in the DTH model of MS:

| | Vehicle | Compound |
|---|---|---|
| Blood brain barrier leakage | 6.325±1.953 | 2.042±0.487 |
| T cell infiltration | 851.1±66.5 | 341.2±21.9 |
| MHC class II expression | 1.763±0.370 | 1.318±0.300 |
| Demyelination | 0.960±0.329 | 0.758±0.227 |

Values are mean ± standard error of the mean

## Claims

1. N¹-[2,2-dimethyl-1S-(pyridin-2-ylcarbamoyl)-propyl]-N⁴-hydroxy-2R-isobutyl-3S-methoxy-succinamide, of formula (II) 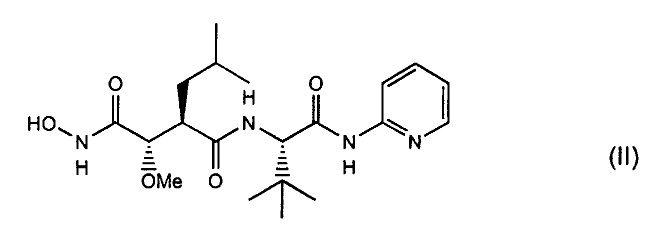 or a pharmaceutically acceptable salt, hydrate or solvate thereof.

2. A pharmaceutical composition comprising a compound as claimed in claim 1 together with a pharmaceutically acceptable carrier.

3. A composition as claimed in claim 2 which is adapted for oral administration.

4. The use of a compound as claimed in claim 1 in the preparation of a pharmaceutical composition for treatment of rheumatoid arthritis, cancers, multiple sclerosis, Guillain-Barre syndrome or psoriasis in mammals, including humans.

## Patentansprüche

1. N¹-[2,2-Dimethyl-1S-(pyridin-2-ylcarbamoyl)-propyl]-N⁴-hydroxy-2R-isobutyl-3S-methoxy-succinamid der Formel (II) : 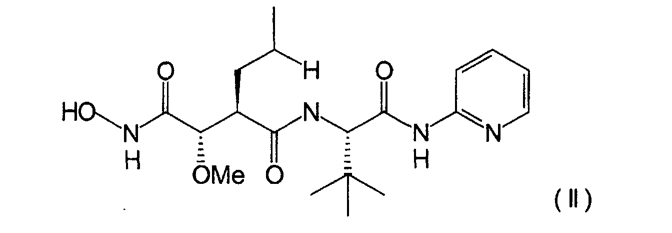 oder ein pharmazeutisch annehmbares Salz, Hydrat oder Solvat davon.

2. Pharmazeutische Zusammensetzung, umfassend eine Verbindung gemäss Anspruch 1 zusammen mit einem pharmazeutisch annehmbaren Träger.

3. Zusammensetzung gemäss Anspruch 2, die zur oralen Verabreichung angepasst ist.

4. Verwendung einer Verbindung gemäss Anspruch 1 bei der Herstellung einer pharmazeutischen Zusammensetzung zur Behandlung von rheumatoider Arthritis, Krebs, Multiple Sklerose, Guillan-Barré-Syndrom oder Psoriasis in Säugern, einschliesslich Menschen.

## Revendications

1. N¹-[2,2-diméthyl-1S-(pyridin-2-ylcarbamoyl)-propyl]-N⁴-hydroxy-2R-isobutyl-3 S-méthoxy-succinamide de formule (II) : 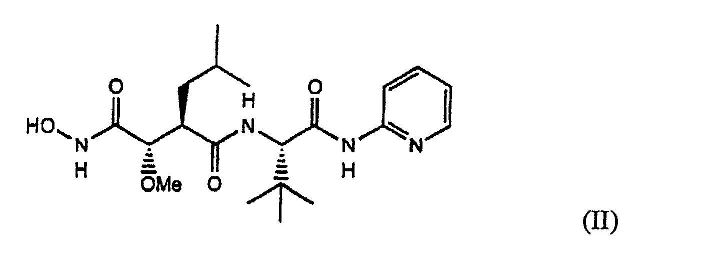 ou un de ses sels, hydrates ou solvates pharmaceutiquement acceptables.

2. Composition pharmaceutique comprenant un composé selon la revendication 1 conjointement avec un véhicule pharmaceutiquement acceptable.

3. Composition selon la revendication 2 qui est adaptée à l'administration orale.

4. Utilisation d'un composé selon la revendication 1 dans la préparation d'une composition pharmaceutique pour le traitement de l'arthrite rhumatoïde, de cancers, de sclérose multiple, du syndrome de Guillain-Barre ou du psoriasis chez les mammifères, y compris les humains.
